# EUROPEAN PATENT APPLICATION

(11) **EP 3 733 057 A1**
(43) Date of publication of application: **04.11.2020**
(21) Application number: 20728375.5
(22) Date of filing: 18.03.2020
(51) Int. Cl.: A61B 5/00, A61B 5/11

(54) **WEARABLE DEVICE, SIGNAL PROCESSING METHOD AND DEVICE**

(30) Priority: 20.03.2019 CN 201910211438
(71) Applicant: Anhui Huami Information Technology Co., Ltd., Hefei, Anhui 230088 (CN)
(72) Inventor: WANG, Kongqiao, Hefei, Anhui 230088 (CN); ZHAO, Mingxi, Hefei, Anhui 230088 (CN); FENG, Di, Hefei, Anhui 230088 (CN); ZHAO, Wei, Hefei, Anhui 230088 (CN); ZHU, Guokang, Hefei, Anhui 230088 (CN)
(74) Representative: Papa, Elisabetta
(86) International application number: PCT/CN2020/080018
(87) International publication number: WO 2020/187266

(57) **Abstract**

The present disclosure provides a wearable device, a signal processing method and a signal processing apparatus, the signal processing method is applied to the wearable device, the wearable device is provided with a biosensor and a motion detector group, and the signal processing method includes the following steps. The human physiological signal collected by the biosensor is acquired, the human physiological signal is divided into multiple signal frames, each of the multiple signal frames corresponds to the time range; for each signal frame, it is determined whether the user wearing the wearable device is in the body motion state according to the body motion signal collected by the motion detector group within the time range corresponding to the signal frame, and when the user wearing the wearable device is not in the body motion state, the signal frame is stored into the preset buffer. Therefore, the body motion by may be sensed by using the body motion signal acquired by the motion detector group to filter the human physiological signal acquired by the biosensor, which may ensure that the filtered human physiological signal is the signal of the user wearing the wearable device in the resting state, thereby improving the stability and reliability of the wearable device in healthcare monitoring and diagnosis, and reducing the false alarm rate.

## Description

### CROSS-REFERENCE TO RELATED APPLICATIONS

The present disclosure claims priority to and benefits of Chinese Patent Application Serial No. 201910211438.7, filed on March 20, 2019, the entire content of which are incorporated herein by reference.

### FIELD

The present disclosure relates to the field of signal processing technology, and more particularly, to a signal processing method, a signal processing apparatus, and a wearable device.

### BACKGROUND

With the development of intelligent hardware, it is more and more widely to acquire physiological signals of human body through biosensors on wearable devices (such as bracelets, watches, etc.) for healthcare monitoring and diagnosis. However, the physiological signals collected by biosensors are usually weak and often interfered by various noises, such as motion noise caused by human motion, contact noise between the sensor and skin, and the like, these noises will directly lead to a decrease in detection performance, and even seriously, the signal will be completely submerged in the noise and cannot be reconstructed, which results in a detection failure. Since the detection requirement of the physiological signal is relatively high, any minor error will bring a negative psychological burden to the user, and thus the reliability of the physiological signal needs to be ensured.

### SUMMARY

The present disclosure seeks to solve at least one of problems existing in the related art to at least some extent. Accordingly, a first objective of the present disclosure is to provide a signal processing method to solve the problem of low reliability of physiological signals acquired by current wearable devices.

A second objective of the present disclosure is to provide a signal processing apparatus.

A third objective of the present disclosure is to provide a wearable device.

In order to achieve the above objective, embodiments of a first aspect of the present disclosure provide a signal processing method, applied to a wearable device provided with a biosensor and a motion detector group, the method includes: acquiring a human physiological signal collected by the biosensor, and dividing the human physiological signal into multiple signal frames, each signal frame corresponds to a time range; for each signal frame, determining whether a user wearing a wearable device is in a body motion state according to a body motion signal collected by the motion detector group within the time range corresponding to the signal frame; and storing the signal frame into a preset buffer when the user wearing the wearable device is not in the body motion state.

According to the signal processing method according to embodiments of the present disclosure, the human physiological signal collected by the biosensor is acquired first, the human physiological signal is divided into multiple signal frames, each signal frame corresponds to the time range, and for each signal frame, it is determined whether the user wearing the wearable device is in the body motion state according to the body motion signal collected by the motion detector group within the time range corresponding to the signal frame, and when the user wearing the wearable device is not in the body motion state, the signal frame is stored into the preset buffer. Thus, with the method, the body motion may be sensed by using the body motion signal acquired by the motion detector group to filter the human physiological signal acquired by the biosensor, which may ensure that the filtered human physiological signal is the signal of the user wearing the wearable device in the resting state, thereby improving the stability and reliability of the wearable device in healthcare monitoring and diagnosis, and reducing the false alarm rate.

In addition, the signal processing method according to the above-mentioned embodiments of the present disclosure may also have the following additional technical features.

According to an embodiment of the present disclosure, the motion detector group includes an inertial sensor, an electromyographic sensor, and a microphone. Determining whether the user wearing the wearing wearable device is in the body motion state according to the body motion signal collected by the motion detector group within the time range corresponding to the signal frame includes: determining whether the user wearing the wearable device has a body motion according to a motion signal collected by the inertial sensor within the time range corresponding to the signal frame; determining whether the user wearing the wearable device has the body motion according to a surface electromyographic signal collected by the electromyographic sensor within the time range corresponding to the signal frame, when determining, according to the motion signal collected by the inertial sensor, that the user wearing the wearable device does not have the body motion; determining whether the user wearing the wearable device has the body motion according to a sound signal between skin and the wearable device collected by the microphone within the time range corresponding to the signal frame, when determining, according to the surface electromyographic signal collected by the electromyographic sensor, that the user wearing the wearable device does not have the body motion; and determining that the user wearing the wearable device is in the body motion state, when determining that the user wearing the wearable device has the body motion according to the signal collected by any one of the inertial sensor, the electromyographic sensor, and the microphone.

According to an embodiment of the present disclosure, determining whether the user wearing the wearable device has the body motion according to the motion signal acquired by the inertial sensor within the time range corresponding to the signal frame includes: acquiring a first low threshold and a first high threshold, in which the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold; determining an activity amount of the user wearing the wearable device according to the motion signal; determining whether there is a signal frame having the activity amount greater than the first high threshold in m signal frames before the signal frame, when the activity amount is less than the first low threshold; and determining that the user wearing the wearable device has the body motion, when there is the signal frame having the activity amount greater than the first high threshold or the activity amount is greater than the first low threshold.

According to an embodiment of the present disclosure, the time range corresponding to the signal frame includes motion signals at multiple moments; determining the activity amount of the user wearing the wearable device according to the motion signal includes: for the motion signal at each moment, performing a differential processing on the motion signal and motion signals at first n moments to obtain a differential value at each moment; and determining an average value of differential values at multiple moments as the activity amount of the user wearing the wearable device.

According to an embodiment of the present disclosure, the time range corresponding to the signal frame includes surface electromyographic signals at multiple moments. Determining whether the user wearing the wearable device has the body motion according to the surface electromyographic signal acquired by the electromyographic sensor within the time range corresponding to the signal frame includes: acquiring a second low threshold and a second high threshold, in which the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold; determining a maximum surface electromyographic signal from the surface electromyographic signals at multiple moments, and obtaining an average surface electromyographic signal of the surface electromyographic signals at multiple moments; and determining that the user wearing the wearable device has the body motion, when the maximum surface electromyographic signal is greater than the second high threshold and the average surface electromyographic signal is greater than a second low threshold.

In order to achieve the above objectives, embodiments of a second aspect of the present disclosure provide a signal processing apparatus, applied to a wearable device provided with a biosensor and a motion detector group, and the apparatus includes an acquisition module, a determining module and a storage module. The acquisition module is configured to acquire a human physiological signal collected by the biosensor, divide the human physiological signal into multiple signal frames, and acquire a body motion signal collected by the motion detector group. Each signal frame corresponds to a time range. The determining module is configured to, for each signal frame, determine whether a user wearing the wearable device is in a body motion state according to the body motion signal collected by the motion detector group within the time range corresponding to the signal frame. The storage module is configured to store the signal frame into a preset buffer when the user wearing the wearable device is not in the body motion state.

According to the signal processing apparatus according to embodiments of the present disclosure, the human physiological signal collected by the biosensor is acquired through the acquisition module, the human physiological signal is divided into multiple signal frames, each signal frame corresponds to the time range, and the body motion signal collected by the motion detector group is acquired, for each signal frame, it is determined whether the user wearing the wearable device is in the body motion state by the determining module according to the body motion signal collected by the motion detector group within the time range corresponding to the signal frame, and the signal frame is stored into the preset buffer by the storage module when the user wearing the wearable device is not in the body motion state. Therefore, with the apparatus, the body motion may be sensed by using the body motion signal acquired by the motion detector group to filter the human physiological signal acquired by the biosensor, which may ensure that the filtered human physiological signal is the signal of the user wearing the wearable device in the resting state, thereby improving the stability and the reliability of the wearable device in the healthcare monitoring and diagnosis, and reducing the false alarm rate.

In addition, the signal processing apparatus according to the above-mentioned embodiments of the present disclosure may have the following additional technical features.

According to an embodiment of the present disclosure, the motion detector group includes an inertial sensor, an electromyographic sensor, and a microphone. The determining module is configured to determine whether the user wearing the wearable device has a body motion according to a motion signal collected by the inertial sensor within the time range corresponding to the signal frame; determine whether the user wearing the wearable device has the body motion according to a surface electromyographic signal collected by the electromyographic sensor within the time range corresponding to the signal frame, when determining, according to the motion signal collected by the inertial sensor, that the user wearing the wearable device does not have the body motion; determine whether the user wearing the wearable device has the body motion according to a sound signal between skin and the wearable device collected by the microphone within the time range corresponding to the signal frame, when determining, according to the surface electromyographic signal collected by the electromyographic sensor, that the user wearing the wearable device does not have the body motion; and determine that the user wearing the wearable device is in the body motion state, when determining, according to the signal collected by any one of the inertial sensor, the electromyographic sensor, and the microphone, that the user wearing the wearable device has the body motion.

According to an embodiment of the present disclosure, in determining whether the user wearing the wearable device has the body motion according to the motion signal acquired by the inertial sensor within the time range corresponding to the signal frame, the determining module is configured to: acquire a first low threshold and a first high threshold, in which the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold; determine an activity amount of the user wearing the wearable device according to the motion signal; determine whether there is a signal frame having the activity amount greater than the first high threshold in m signal frames before the signal frame, when the activity amount is less than the first low threshold; and determine that the user wearing the wearable device has the body motion, when there is the signal frame having the activity amount greater than the first high threshold or the activity amount is greater than the first low threshold.

According to an embodiment of the present disclosure, the time range corresponding to the signal frame includes motion signals at multiple moments. In determining the activity amount of the user wearing the wearable device according to the motion signal, the determining module is configured to: for the motion signal at each moment, perform a differential processing on the motion signal and motion signals at first n moments to obtain a differential value at each of the plurality of moments; and determine an average value of differential values at multiple moments as the activity amount of the user wearing the wearable device.

According to an embodiment of the present disclosure, the time range corresponding to the signal frame includes surface electromyographic signals at multiple moments. In determining whether the user wearing the wearable device has the body motion according to the surface electromyographic signal acquired by the electromyographic sensor within the time range corresponding to the signal frame, the determining module is configured to: acquire a second low threshold and a second high threshold, in which the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold; determine a maximum surface electromyographic signal from the surface electromyographic signals at multiple moments, and obtain an average surface electromyographic signal of the surface electromyographic signals at multiple moments; and determine that the user wearing the wearable device has the body motion, when the maximum surface electromyographic signal is greater than the second high threshold and the average surface electromyographic signal is greater than a second low threshold.

In order to achieve the above objective, embodiments of a third aspect of the present disclosure provide a wearable device, the wearable device includes a readable storage medium and a processor. The readable storage medium is configured to store machine executable instructions, the processor is configured to read the machine executable instructions stored in the readable storage medium and execute the instructions to implement the method of the first aspect.

By applying the embodiments of the present disclosure, when the human physiological signal collected by the biosensor arranged on the wearable device is acquired, the human physiological signal is divided into multiple signal frames, each signal frame corresponds to the time range, and for each signal frame, it is determined whether the user wearing the wearable device is in the body motion state according to the body motion signal collected by the motion detector group arranged on the wearable device within the time range corresponding to the signal frame, and when the user wearing the wearable device is not in the body motion state, the signal frame is stored into the preset buffer.

Based on the description, the body motion is sensed by using the body motion signal acquired by the motion detector group, and the human physiological signal acquired by the biosensor is filtered, so as to ensure that the filtered human physiological signal is the signal of the user wearing the wearable device in the resting state, thereby improving the stability and reliability of the wearable device in healthcare monitoring and diagnosis, and reducing the false alarm rate.

Additional aspects and advantages of the present disclosure will be given in part in the following descriptions, become apparent in part from the following descriptions, or be learned from the practice of the present disclosure.

### BRIEF DESCRIPTION OF THE DRAWINGS

The above and/or additional aspects and advantages of embodiments of the present disclosure will become apparent and more readily appreciated from the following descriptions made with reference to the drawings, in which:
FIG. 1 is a schematic diagram of PhotoPlethysmoGraphy (PPG) signals according to some exemplary embodiments of the present disclosure;
FIG. 2A is a flowchart illustrating a signal processing method according to some exemplary embodiments of the present disclosure;
FIG. 2B is a schematic diagram illustrating a signal frame division of a human physiological signal according to embodiments of FIG. 2A;
FIG. 2C is a schematic diagram illustrating a surface electromyographic signal according to embodiments of FIG. 2A;
FIG. 3 is a schematic diagram illustrating a hardware structure of a wearable device according to some exemplary embodiments of the present disclosure; and
FIG. 4 is a block diagram illustrating a signal processing apparatus according to some exemplary embodiments of the present disclosure.

### DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the present disclosure. Instead, they are merely examples of apparatuses and methods consistent with aspects related to the present disclosure as recited in the appended claims.

The terms used herein is for the purpose of describing particular embodiments only and is not intended to be limiting of the present disclosure. As used in the present disclosure and the appended claims, singular forms "a", "an", and "the" are intended to include plural forms as well, unless the context clearly indicates otherwise. It should also be understood that, the term "and/or" as used herein refers to and encompasses any and all possible combinations of one or more of the associated listed items.

It should be understood that, although the terms first, second, third, etc. may be used herein to describe various information, such information should not be limited to these terms. These terms are only used to distinguish one type of information from another. For example, first information may also be referred to as second information, and similarly, the second information may also be referred to as the first information, without departing from the scope of the present disclosure. The word "if' as used herein, may be interpreted as "at......" or "when......" or "in response to a determination", depending on the context.

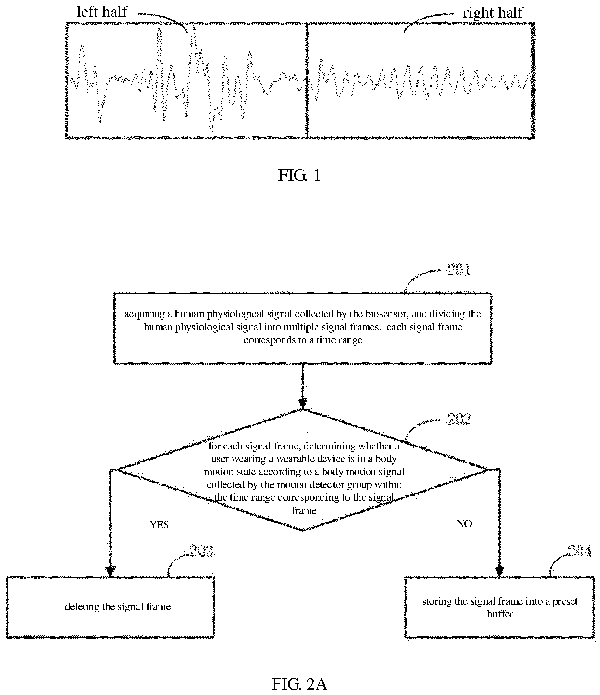

At present, biosensors on wearable devices (such as bracelets, watches, etc.) are used to acquire human physiological signals to perform healthcare monitoring and diagnosis, such as heart rate detection and cardiovascular disease diagnosis based on PPG signal or ECG (electrocardiogram) signals acquired by the biosensor. FIG. 1 is schematic diagram of PPG signals according to some exemplary embodiments of the present disclosure, the PPG signals in the right half of FIG. 1 are regular, and belong to normal signals, and the PPG signals in the left half are irregular, and belong to noise signals (actually caused by the motion of the user). When the PPG signal in the left half of FIG. 1 is used for heart rate detection, the detected heart rate value may be far different from the actual heart rate value of the user, which results in a high false alarm rate.

However, from a detection perspective, as long as the user wearing the wearable device is ensured to perform physiological signal acquisition in a resting state, noise interference may be avoided, and the reliability of the physiological disease diagnosis by using the wearable device can be ensured.

Based on this, the present disclosure provides a signal processing method, when the human physiological signal collected by the biosensor arranged on the wearable device is acquired, the human physiological signal is divided into multiple signal frames, each signal frame corresponds to a time range, and for each signal frame, it is determined whether the user wearing the wearable device is in a body motion state according to the body motion signal collected by the motion detector group arranged on the wearable device within the time range corresponding to the signal frame, and the signal frame is stored into the preset buffer when the user wearing the wearable device is not in the body motion state.

Based on the description, the body motion is sensed by using the body motion signal acquired by the motion detector group, and the human physiological signal acquired by the biosensor is filtered, so as to ensure that the filtered human physiological signal is the signal of the user wearing the wearable device in the resting state, thereby improving the stability and reliability of the wearable device in healthcare monitoring and diagnosis, and reducing the false alarm rate.

The signal processing method according to the present disclosure will be described in detail below with specific embodiments.

FIG. 2A is a flowchart illustrating a signal processing method according to some exemplary embodiments of the present disclosure. The signal processing method may be applied to a wearable device provided with the biosensor and the motion detector group, the biosensor may include a PPG sensor, a pressure sensor, an ECG sensor, and the motion detector group may include an inertial sensor (e.g., an accelerometer, a gyroscope, and the like), an electromyographic sensor, a microphone, and the like.

As shown in FIG. 2A, the signal processing method includes following steps.

At block 201, the human physiological signal collected by the biosensor is acquired, and the human physiological signal is divided into multiple signal frames, and each signal frame corresponds to a time range.

In some embodiments, the human physiological signal collected by the biosensor in a preset time period may be acquired, and then the human physiological signal acquired may be divided into multiple signal frames with the preset time period as a division period.

Illustratively, the division manner may be an overlapping division manner, and certainly, a non-overlapping division manner may also be adopted, which is not limited in the present disclosure. For the overlapping division manner, the overlapping ratio between signal frames may be set according to practical experience.

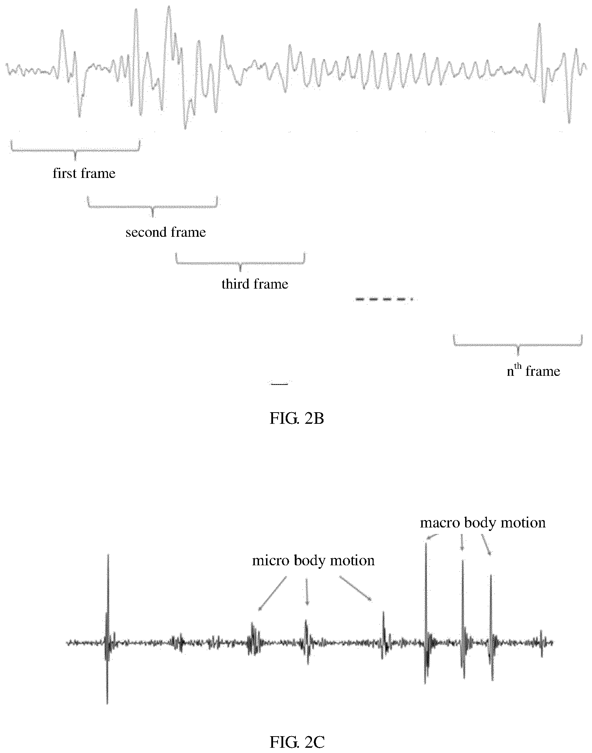

For a segment of PPG signals illustrated in FIG. 2B, the PPG signal may be divided in an overlapping division manner to obtain n signal frames, and the overlapping ratio between adjacent signal frames is 50%.

At block 202, for each signal frame, it is determined whether the user wearing the wearable device is in a body motion state according to the body motion signal collected by the motion detector group within the time range corresponding to the signal frame, if so, block 203 is executed, and otherwise, block 204 is executed.

In some embodiments, in the case where the motion detector group includes the inertial sensor, the electromyographic sensor, and the microphone, it may first be determined whether the user wearing the wearable device has the body motion according to the motion signal collected by the inertial sensor within the time range corresponding to the signal frame, when it is determined, according to the motion signal collected by the inertial sensor, that the user wearing the wearable device does not have the body motion, it is further determined whether the user wearing the wearable device has the body motion according to a surface electromyographic signal collected by the electromyographic sensor within the time range corresponding to the signal frame, when it is determined, according to the surface electromyographic signal collected by the electromyographic sensor, that the user wearing the wearable device does not have the body motion, it is further determined whether the user wearing the wearable device has the body motion according to a sound signal between skin and the wearable device collected by the microphone within the time range corresponding to the signal frame, when it is determined that the user wearing the wearable device has the body motion according to the signal collected by any one of the inertial sensor, the electromyographic sensor, and the microphone, it is determined that the user wearing the wearable device is in the body motion state.

It should be noted that, the body motion of the user may be divided into two types: macro body motion and micro body motion. The macro body motion refers to obvious physical motion of the user in space, and the physical motion can be obviously perceived, such as arm raising, arm swinging and the like, and the activity amount can be detected for judgment, based on the motion signal acquired by the inertial sensor (such as the accelerometer, the gyroscope and the like). The micro body motion refers to motion that are not easily perceived by naked eyes, such as muscle tension caused by tiny wrist movement, friction between the device and the skin caused by device sideslip due to loosen wearing, etc. Limited by the sensitivity of the inertial sensor, it is difficult to detect the activity amount through the inertial sensor, or the activity amount is submerged in the noise because it is too small, the muscle tension can be detected for judgment based on the surface electromyographic (sEMG) signal collected by the electromyographic sensor, or the friction noise between the device and the skin can be listened for judgment based on the sound signal collected by the microphone near the skin.

Thus, by combining body motion parameters detected by multiple motion detectors, comprehensive judgment of the body motion state can be achieved, such that both macro body motion and micro body motion can be accurately detected.

Based on the above analysis, it is easy to use the inertial sensor to determine macro body motion, the body motion state is first determined according to the motion signal collected by the inertial sensor, when there is no body motion, the body motion state is further determined according to the surface electromyographic signal collected by the electromyographic sensor, and when there is no body motion, the body motion state is further determined according to the sound signal collected by the microphone, and finally, when it is determined that the user wearing the wearable device have no body motion through the three motion detectors, it is determined that the user wearing the wearable device is in the resting state. Certainly, other determination sequences may also be adopted, for example, it may be determined first by electromyographic sensor, then the inertial sensor, and then the microphone.

It should be understood by those skilled in the art that, in addition to the above-described inertial sensor, the electromyographic sensor, and the microphone, other motion detectors for detecting the body motion, such as the brain wave sensor, may be used.

In some embodiments, determining whether the user wearing the wearable device has the body motion according to the motion signal acquired by the inertial sensor within the time range corresponding to the signal frame may include acts of: acquiring a first low threshold and a first high threshold, in which the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold; determining an activity amount of the user wearing the wearable device according to the motion signal; determining whether there is a signal frame having the activity amount greater than the first high threshold in m signal frames before the signal frame, when the activity amount is less than the first low threshold; and determining that the user wearing the wearable device has the body motion, when there is the signal frame having the activity amount greater than the first high threshold or the activity amount is greater than the first low threshold. m may be adjusted according to experience.

The local characteristics of the current signal frame are considered when determining that activity amount of the current signal frame is greater than the first low threshold, and the global characteristics of the m signal frames before the current signal frame are considered when determining the m signal frames before the current signal frame, the local characteristics and the global characteristics of the body motion are considered through the two determinations, making the judgment result more effective and accurate.

In a possible implementation, the first low threshold and the first high threshold may be obtained in advance from data historically collected, to facilitate subsequent use. Assuming that the motion signals are framed according to a large number of motion signals collected in the resting state in history, and the average value of the activity amount is obtained as ACT1, and the motion signals are framed according to a large number of motion signals collected in the body motion state in history, and the average value of the activity amount is obtained as ACT2, ACT1+ deltal may be taken as the first low threshold Thre1, and ACT2-delta2 may be taken as the first high threshold Thre2. delta1 and delta2 represent the disturbance value in the resting state and the disturbance value in the body motion state, respectively, and delta1 and delta2 may be obtained by machine learning methods such as the decision tree and SVM (Support Vector Machine), and may also be obtained by experience.

In some embodiments, the motion signal collected by the inertial sensor within the time range corresponding to the signal frame includes motion signals at multiple moments. Based on this, determining the activity amount of the user wearing the wearable device according to the motion signal may include acts of: for the motion signal at each moment, performing a differential processing on the motion signal and motion signals at first n moments to obtain a differential value at each moment; and determining an average value of differential values at multiple moments as the activity amount of the user wearing the wearable device.

In a possible implementation, when the inertial sensor is an acceleration sensor, the motion signal at each moment includes acceleration values in three directions. One way to calculate the activity amount of the current signal frame may include the followings. The differential processing is performed on the acceleration values in the three directions at each moment in the current frame and the acceleration values in the three directions at first n moments to obtain differential values in the three directions, and then an average value of the differential values in each direction is determined, and the maximum average value is determined from the average values in the three directions as the activity amount of the user wearing the wearable device. Another way to calculate the activity amount of the current signal frame may include the followings. The modulus of the acceleration values in the three directions at each multiple moment are determined, the differential processing is performed on the modulus at each moment and the modulus at the first n moments, and the average value of the differential values at multiple moments is determined as the activity amount of the user wearing the wearable device.

In another possible implementation, when the inertial sensor is a gyro sensor, the motion signal at each moment may include rotational angular velocity values in three directions. A calculation method of the activity amount of the current signal frame may include the followings. The rotational angular velocity values in the three directions at each moment and the rotational angular velocity values in the three directions at the first n moments are subjected to the differential processing to obtain the differential values in the three directions, and then an average value of the differential values in each direction is determined, and the maximum average value is determined from the average values in the three directions as the activity amount of the user wearing the wearable device.

It should be noted that, in order to strictly filter the signal frames, the maximum average value may be selected from the average values in the three directions as the activity amount of the user wearing the wearable device. It will be understood by those skilled in the art that, the average value of the average values of the three directions may also be determined as the activity amount of the user wearing the wearable device, or a minimum average value may be selected from the average values in the three directions as the activity amount of the user wearing the wearable device.

In some embodiments, the time range corresponding to the signal frame includes surface electromyographic signals at multiple moments, based on this, determining whether the user wearing the wearable device has the body motion according to the surface electromyographic signal acquired by the electromyographic sensor within the time range corresponding to the signal frame may include acts of: acquiring a second low threshold and a second high threshold, in which the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold; determining a maximum surface electromyographic signal from the surface electromyographic signals at multiple moments, and obtaining an average surface electromyographic signal of the surface electromyographic signals at multiple moments; and determining that the user wearing the wearable device has the body motion when the maximum surface electromyographic signal is greater than the second high threshold and the average surface electromyographic signal is greater than a second low threshold.

The surface electromyographic signal is a non-stationary weak signal, which can reflect the motion state of corresponding skeletal muscles, and is formed by superposing various action potential sequences on the skin surface, the various action potential sequences are formed by continuous action potential generated by muscle fiber membrane, which is caused by continuous transmission of nerve impulses to nerve endings by the central nervous cells of the human body. As shown in FIG. 2C, when the user is in the resting state, the surface electromyographic signal is composed of weak noises caused by weak motions, and when the palm and fingers have large movements, the surface electromyographic signal has obvious fluctuation. Thus, the body motion condition of the user may be accurately determined by using the surface electromyographic signal, ensuing that the filtered human physiological signal are signals of the user wearing the wearable device in the resting state, and the misjudgment caused by some abnormal interferences may be avoided by suing the judgment condition of the second low threshold and the second high threshold.

Illustratively, the second low threshold and the second high threshold may also be obtained in advance according to data collected in history for subsequent use, and the obtaining principle thereof may be the same as that of the first low threshold and the first high threshold, and will not be described in detail herein.

Illustratively, before the body motion is determined, the surface electromyographic signals acquired by the electromyographic sensor may be filtered to remove low-frequency baseline drift and 50Hz power frequency interference noise. Butterworth filter can be used for filtering.

In some embodiments, for the process of determining whether the user wearing the wearable device has the body motion according to the sound signal collected by the microphone between the skin and the wearable device within the time range corresponding to the signal frame, since the sound signal represents the noise level generated by the friction between the device and the skin, and the surface electromyographic signal represents the noise level generated by muscle tension, the principle of determining whether the user wearing the wearable device has the body motion according to the sound signal may be the same as the principle of determining whether the user wearing the wearable device has the body motion according to the surface electromyographic signal, and the present disclosure is not described in detail.

At block 203, the signal frame is deleted.

At block 204, the signal frame is stored into a preset buffer.

Based on the division manner of the signal frame described in the above block 201, for the overlapping division manner, when it is determined that a certain signal frame is acquired in the resting state and stored, it needs to determine whether a previous signal frame of the signal frame is acquired in the resting state, and if not, the signals overlapped between the signal frame and the previous signal frame are deleted, and then remaining signals in the signal frame are stored in the preset buffer.

Further, for the non-overlapping division mode, when it is determined that a signal frame is collected in the resting state, the signal frame is directly stored, and if the signal frame is not collected in the resting state, the signal frame is directly deleted.

Through the above processes from block 201 to block 204, the human physiological signals stored in the preset buffer are all physiological signals of the user wearing the wearable device in the resting state, and may be accurately used for analysis and detection of various physiological signs, such as heart rate, HRV, sleep index, atrial fibrillation, blood oxygen, blood pressure, blood sugar, etc.

In embodiments of the present disclosure, when the human physiological signal collected by the biosensor arranged on the wearable device is acquired, the human physiological signal is divided into multiple signal frames, each signal frame corresponds to the time range, and for each signal frame, it is determined whether the user wearing the wearable device is in a body motion state according to the body motion signal collected by the motion detector group arranged on the wearable device within the time range corresponding to the signal frame, and when the user wearing the wearable device is not in the body motion state, the signal frame is stored into the preset buffer.

Based on the description, the body motion is sensed by using the body motion signal acquired by the motion detector group, and the human physiological signal acquired by the biosensor is filtered, so as to ensure that the filtered human physiological signal is the signal of the user wearing the wearable device in the resting state, thereby improving the stability and reliability of the wearable device in healthcare monitoring and diagnosis, and reducing the false alarm rate.

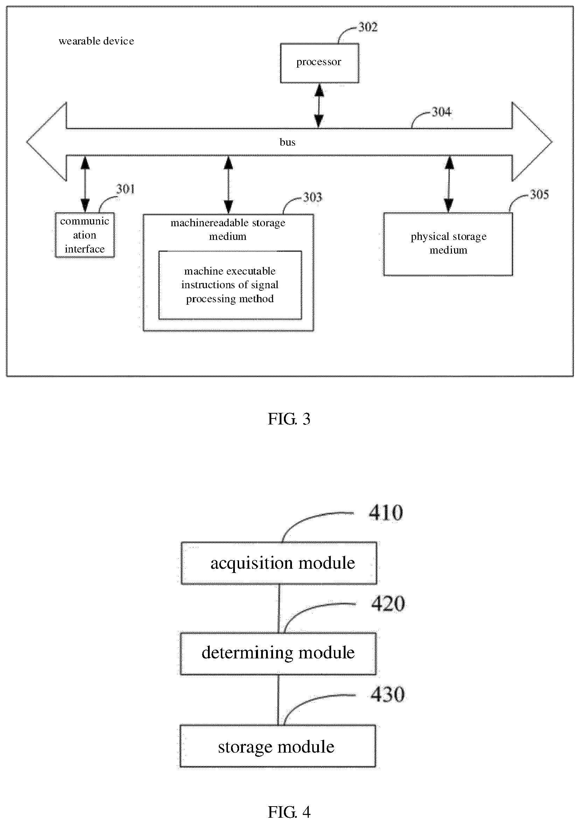

FIG. 3 is a hardware block diagram illustrating a wearable device according to some exemplary embodiments of the present disclosure. The wearable device includes a communication interface 301, a processor 302, a machine-readable storage medium 303, and a bus 304; the communication interface 301, the processor 302, and the machine-readable storage medium 303 communicate with each other via the bus 304. The processor 302 may execute the signal processing method described above by reading and executing machine executable instructions corresponding to the control logic of the signal processing method in the machine readable storage medium 303, and the specific content of the method is referred to the above embodiments, which will not be elaborated here.

The machine-readable storage medium 303 referred to herein may be any electronic, magnetic, optical, or other physical storage device that can contain or store information such as executable instructions, data, and the like. For example, the machine-readable storage medium may be volatile memory, non-volatile memory, or similar storage media. In particular, the machine-readable storage medium 303 may be a RAM (random Access Memory), a flash Memory, a storage drive (e.g., a hard drive), any type of storage disk (e.g., an optical disk, a DVD, etc.), or similar storage medium, or a combination thereof.

FIG. 4 is a block diagram illustrating a signal processing apparatus according to some exemplary embodiments of the present disclosure. The signal processing apparatus may be applied to a wearable device provided with the biosensor and the motion detector group, and the signal processing apparatus includes an acquisition module 410, a determining module 420 and a storage module 430.

The acquisition module 410 is configured to acquire the human physiological signal collected by the biosensor, divide the human physiological signal into multiple signal frames, and acquire the body motion signal collected by the motion detector group. Each signal frame corresponds to the time range.

The determining module 420 is configured to, for each signal frame, determine whether the user wearing the wearable device is in the body motion state according to the body motion signal collected by the motion detector group within the time range corresponding to the signal frame.

The storage module 430 is configured to store the signal frame into the preset buffer when the user wearing the wearable device is not in the body motion state.

In an optional implementation, the motion detector group includes the inertial sensor, the electromyographic sensor, and the microphone. The determining module 420 is further configured to: determine whether the user wearing the wearable device has a body motion according to a motion signal collected by the inertial sensor within the time range corresponding to the signal frame; determine whether the user wearing the wearable device has the body motion according to a surface electromyographic signal collected by the electromyographic sensor within the time range corresponding to the signal frame, when determining, according to the motion signal collected by the inertial sensor, that the user wearing the wearable device does not have the body motion; determine whether the user wearing the wearable device has the body motion according to a sound signal between skin and the wearable device collected by the microphone within the time range corresponding to the signal frame, when determining, according to the surface electromyographic signal collected by the electromyographic sensor, that the user wearing the wearable device does not have the body motion; and determine that the user wearing the wearable device is in the body motion state, when determining, according to the signal collected by any one of the inertial sensor, the electromyographic sensor, and the microphone, that the user wearing the wearable device has the body motion.

In an optional implementation, in determining whether the user wearing the wearable device has the body motion according to the motion signal acquired by the inertial sensor within the time range corresponding to the signal frame, the determining module 420 is configured to: acquire a first low threshold and a first high threshold, in which the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold; determine an activity amount of the user wearing the wearable device according to the motion signal; determine whether there is a signal frame having the activity amount greater than the first high threshold in m signal frames before the signal frame, when the activity amount is less than the first low threshold; and determine that the user wearing the wearable device has the body motion, when there is the signal frame having the activity amount greater than the first high threshold or the activity amount is greater than the first low threshold.

In an optional implementation, the time range corresponding to the signal frame includes the motion signals at multiple moments. In determining the activity amount of the user wearing the wearable device according to the motion signal, the determining module 420 is configured to: for the motion signal at each moment, perform a differential processing on the motion signal and motion signals at first n moments to obtain a differential value at each moment; and determine an average value of differential values at multiple moments as the activity amount of the user wearing the wearable device.

In an optional implementation, the time range corresponding to the signal frame includes surface electromyographic signals at multiple moments. In determining whether the user wearing the wearable device has the body motion according to the surface electromyographic signal acquired by the electromyographic sensor within the time range corresponding to the signal frame, the determining module 420 is configured to: acquire a second low threshold and a second high threshold, in which the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold; determine a maximum surface electromyographic signal from the surface electromyographic signals at multiple moments, and obtain an average surface electromyographic signal of the surface electromyographic signals at multiple moments; and determine that the user wearing the wearable device has the body motion, when the maximum surface electromyographic signal is greater than the second high threshold and the average surface electromyographic signal is greater than a second low threshold.

The specific details of the implementation process of the functions and actions of each unit in the above apparatus are the same as the implementation processes of the corresponding steps in the above method, which will not be elaborated here.

For the apparatus embodiments, since it basically corresponds to the method embodiment, reference may be made to the partial description of the method embodiment for relevant points. The above-described embodiments of the apparatus are merely illustrative, and the units described as separate parts may or may not be physically separate, and parts displayed as units may or may not be physical units, may be located in one position, or may be distributed on multiple network units. Some or all of the modules may be selected according to actual needs to achieve the purpose of the scheme of the present disclosure. One of ordinary skill in the art may understand and implement without inventive effort.

Other embodiments of the present disclosure will be apparent to those skilled in the art from consideration of the specification and practice of the invention disclosed herein. the present disclosure is intended to cover any variations, uses, or adaptations of the invention following, in general, the principles of the present disclosure and including such departures from the present disclosure as come within known or customary practice within the art to which the invention pertains. It is intended that the specification and examples be considered as exemplary only, with a true scope and spirit of the present disclosure being indicated by the following claims.

It should also be noted that the terms "include", "contain", or any other variation thereof, are intended to cover a non-exclusive inclusion, such that a process, method, article, or apparatus that includes a list of elements does not include only those elements but may include other elements not expressly listed or inherent to such process, method, article, or apparatus. Without further limitation, an element defined by the phrase "include one ..." does not exclude the presence of other identical elements in the process, method, article, or apparatus that includes the element.

The above description is only preferred embodiments of the present disclosure and should not be taken as limiting the present disclosure, and any modifications, equivalents, improvements and the like made within the spirit and principle of the present disclosure should be included in the protection scope of the present disclosure.

## Claims

1. A signal processing method, applied to a wearable device provided with a biosensor and a motion detector group, the method comprising:
acquiring a human physiological signal collected by the biosensor, and dividing the human physiological signal into a plurality of signal frames, wherein each of the plurality of signal frames corresponds to a time range;
for each of the plurality of signal frames, determining whether a user wearing a wearable device is in a body motion state according to a body motion signal collected by the motion detector group within the time range corresponding to the signal frame; and
storing the signal frame into a preset buffer when the user wearing the wearable device is not in the body motion state.

2. The method of claim 1, wherein the motion detector group comprises an inertial sensor, an electromyographic sensor, and a microphone;
determining whether the user wearing the wearing wearable device is in the body motion state according to the body motion signal collected by the motion detector group within the time range corresponding to the signal frame comprises:
determining whether the user wearing the wearable device has a body motion according to a motion signal collected by the inertial sensor within the time range corresponding to the signal frame;
determining whether the user wearing the wearable device has the body motion according to a surface electromyographic signal collected by the electromyographic sensor within the time range corresponding to the signal frame, when determining, according to the motion signal collected by the inertial sensor, that the user wearing the wearable device does not have the body motion;
determining whether the user wearing the wearable device has the body motion according to a sound signal between skin and the wearable device collected by the microphone within the time range corresponding to the signal frame, when determining, according to the surface electromyographic signal collected by the electromyographic sensor, that the user wearing the wearable device does not have the body motion; and
determining that the user wearing the wearable device is in the body motion state, when determining that the user wearing the wearable device has the body motion according to the signal collected by any one of the inertial sensor, the electromyographic sensor, and the microphone.

3. The method of claim 2, wherein determining whether the user wearing the wearable device has the body motion according to the motion signal acquired by the inertial sensor within the time range corresponding to the signal frame comprises:
acquiring a first low threshold and a first high threshold, wherein the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold;
determining an activity amount of the user wearing the wearable device according to the motion signal;
determining whether there is a signal frame having the activity amount greater than the first high threshold in m signal frames before the signal frame, when the activity amount is less than the first low threshold; and
determining that the user wearing the wearable device has the body motion, when there is the signal frame having the activity amount greater than the first high threshold or the activity amount is greater than the first low threshold.

4. The method of claim 3, wherein the time range corresponding to the signal frame comprises motion signals at a plurality of moments;
determining the activity amount of the user wearing the wearable device according to the motion signal comprises:
for the motion signal at each of the plurality of moments, performing a differential processing on the motion signal and motion signals at first n moments to obtain a differential value at each of the plurality of moments; and
determining an average value of differential values at the plurality of moments as the activity amount of the user wearing the wearable device.

5. The method according to claim 2, wherein the time range corresponding to the signal frame comprises surface electromyographic signals at a plurality of moments;
determining whether the user wearing the wearable device has the body motion according to the surface electromyographic signal acquired by the electromyographic sensor within the time range corresponding to the signal frame comprises:
acquiring a second low threshold and a second high threshold, wherein the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold;
determining a maximum surface electromyographic signal from the surface electromyographic signals at the plurality of moments, and obtaining an average surface electromyographic signal of the surface electromyographic signals at the plurality of moments; and
determining that the user wearing the wearable device has the body motion, when the maximum surface electromyographic signal is greater than the second high threshold and the average surface electromyographic signal is greater than a second low threshold.

6. A signal processing apparatus, applied to a wearable device provided with a biosensor and a motion detector group, the apparatus comprising:
an acquisition module, configured to acquire a human physiological signal collected by the biosensor, divide the human physiological signal into a plurality of signal frames, and acquire a body motion signal collected by the motion detector group, wherein each of the plurality of signal frames corresponds to a time range;
a determining module, configured to, for each of the plurality of signal frames, determine whether a user wearing the wearable device is in a body motion state according to the body motion signal collected by the motion detector group within the time range corresponding to the signal frame; and
a storage module, configured to store the signal frame into a preset buffer when the user wearing the wearable device is not in the body motion state.

7. The apparatus of claim 6, wherein the motion detector group comprises an inertial sensor, an electromyographic sensor, and a microphone;
the determining module is further configured to:
determine whether the user wearing the wearable device has a body motion according to a motion signal collected by the inertial sensor within the time range corresponding to the signal frame;
determine whether the user wearing the wearable device has the body motion according to a surface electromyographic signal collected by the electromyographic sensor within the time range corresponding to the signal frame, when determining, according to the motion signal collected by the inertial sensor, that the user wearing the wearable device does not have the body motion;
determine whether the user wearing the wearable device has the body motion according to a sound signal between skin and the wearable device collected by the microphone within the time range corresponding to the signal frame, when determining, according to the surface electromyographic signal collected by the electromyographic sensor, that the user wearing the wearable device does not have the body motion; and
determine that the user wearing the wearable device is in the body motion state, when determining, according to the signal collected by any one of the inertial sensor, the electromyographic sensor, and the microphone, that the user wearing the wearable device has the body motion.

8. The apparatus according to claim 7, wherein in determining whether the user wearing the wearable device has the body motion according to the motion signal acquired by the inertial sensor within the time range corresponding to the signal frame, the determining module is configured to:
acquire a first low threshold and a first high threshold, wherein the first low threshold is acquired according to the motion signal collected by the inertial sensor when the user wearing the wearable device is in a resting state, the first high threshold is acquired according to the motion signal collected by the inertial sensor when the user wearing the wearable device is in the body motion state, and the first low threshold is less than the first high threshold;
determine an activity amount of the user wearing the wearable device according to the motion signal;
determine whether there is a signal frame having the activity amount greater than the first high threshold in m signal frames before the signal frame, when the activity amount is less than the first low threshold; and
determine that the user wearing the wearable device has the body motion, when there is the signal frame having the activity amount greater than the first high threshold or the activity amount is greater than the first low threshold.

9. The apparatus of claim 8, wherein the time range corresponding to the signal frame comprises motion signals at a plurality of moments;
in determining the activity amount of the user wearing the wearable device according to the motion signal, the determining module is configured to:
for the motion signal at each of the plurality of moments, perform a differential processing on the motion signal and motion signals at first n moments to obtain a differential value at each of the plurality of moments; and
determine an average value of differential values at the plurality of moments as the activity amount of the user wearing the wearable device.

10. The apparatus according to claim 7, wherein the time range corresponding to the signal frame comprises surface electromyographic signals at a plurality of moments;
in determining whether the user wearing the wearable device has the body motion according to the surface electromyographic signal acquired by the electromyographic sensor within the time range corresponding to the signal frame, the determining module is configured to:
acquire a second low threshold and a second high threshold, wherein the second low threshold is acquired according to the surface electromyographic signal acquired by the electromyographic sensor when the user wearing the wearable device is in the resting state, the second high threshold is acquired according to the surface electromyographic signal acquired by the electromyographic sensor when the user wearing the wearable device is in the body motion state, and the second low threshold is less than the second high threshold;
determine a maximum surface electromyographic signal from the surface electromyographic signals at the plurality of moments, and obtain an average surface electromyographic signal of the surface electromyographic signals at the plurality of moments; and
determine that the user wearing the wearable device has the body motion, when the maximum surface electromyographic signal is greater than the second high threshold and the average surface electromyographic signal is greater than a second low threshold.

11. A wearable device, comprising:
a readable storage medium, configured to store machine executable instructions; and
a processor;
wherein the processor is configured to read the machine executable instructions stored in the readable storage medium, to execute the instructions to implement the method of any one of claims 1 to 5.
